# NEW EUROPEAN PATENT SPECIFICATION

(11) **EP 3 456 821 B2**
(45) Date of publication and mention of the opposition decision: **24.01.2024**
(45) Mention of the grant of the patent: 12.08.2020
(21) Application number: 17191829.5
(22) Date of filing: 19.09.2017
(51) Int. Cl.: C12N 15/00

(54) **NON-INTEGRATING DNA VECTORS FOR THE GENETIC MODIFICATION OF CELLS**
NICHT INTEGRIERENDE DNA-VEKTOREN ZUR GENETISCHEN MODIFIKATION VON ZELLEN
VECTEURS D'ADN NON INTÉGRANTS DESTINÉS À LA MODIFICATION GÉNÉTIQUE DE CELLULES

(43) Date of publication of application: 20.03.2019
(73) Proprietor: Deutsches Krebsforschungszentrum, 69120 Heidelberg (DE)
(72) Inventor: HARBOTTLE, Richard, 68526 Ladenburg (DE); BOZZA, Matthias, 69121 Heidelberg (DE); WILLIAMS, James A., Lincoln, Nebraska 68516 (US)
(74) Representative: Altmann Stößel Dick Patentanwälte PartG mbB

(56) References cited:
- WO-A1-2014/016580
- WO-A2-2010/018444
- US-A- 5 985 607
- ANJA EHRHARDT ET AL: "Episomal Vectors for Gene Therapy", CURRENT GENE THERAPY, vol. 8, no. 3, 1 June 2008 (2008-06-01), pages 147-161, XP055436780, NL ISSN: 1566-5232, DOI: 10.2174/156652308784746440

## Description

The present disclosure relates to a polynucleotide comprising at least one promoter and an S/MAR element, wherein said S/MAR element is located downstream of said promoter and wherein the nucleic acid sequence of said S/MAR element (S/MAR sequence) comprises at least 3 sequence motifs ATTA (SEQ ID NO:1) per 100 nucleotides over a stretch of at most 200 nucleotides; the present disclosure further relates to a composition and to a host cell comprising said polynucleotide, and to the polynucleotide for use in medicine and for use in treating genetic disease. The present disclosure also relates to a kit and to a device comprising said polynucleotide, and to methods and uses related to the polynucleotide.

Genetic modification of cells is used routinely in modern cell culture for scientific purposes. However, use of corresponding techniques in treatment of inherited diseases caused by mutations of genes, while being highly desirable, still is hampered by the problem that methods available usually only provide transient modification, such as transient transfection protocols, whereas methods providing stable modification of cells usually rely on integration of the transgene into the genome of the host cell. Integration of a transgene, however, even if targeted to a specific locus, bears the risk of inducing a deleterious mutation, which may lead e.g. to cancer as a side effect of treatment.

Scaffold/matrix attachment regions (S/MARs), which are also known as scaffold-attachment regions (SARs) or matrix-associated regions (MARs) are known as sequences in the genome of eukaryotic organisms mediating attachment of the nuclear matrix. The S/MARS are AT-rich sequences, and some AT-rich motifs were found to be further enriched (Liebeich et al. (2002), NAR 30(15): 3433). A variety of vectors has been proposed for stable maintenance in cells based on S/MAR motifs, e.g. in US 6,410,314 B1 and in Haase et al. (2010), BMC Biotechnology 10:20; moreover, epigenetic effects having an influence on replication of such vectors were identified (Haase et al.(2013), PLOS One 8(11):e79262). US 5,985,607 proposed recombinant DNA molecules comprising a nucleic acid molecule encoding mammalian erythropoietin (EPO) or tissue plasminogen activator (tPA), an expression control sequence operatively linked thereto and at least one SAR element for expressing recombinant mammalian EPO or tPA.

There is, nonetheless, a need in the art for improved means and methods for stable transfection of cells, in particular using S/MAR elements and avoiding the risks involved with integration of the transgene into the genome of the host cell. This problem is solved by the means and methods disclosed herein.

Accordingly, the present invention relates to a polynucleotide comprising at least one promoter and an S/MAR element according to claim 1. Further disclosed is a polynucleotide comprising at least one promoter and an S/MAR element, wherein said S/MAR element is located downstream of said promoter and wherein the nucleic acid sequence of said S/MAR element (S/MAR sequence) comprises at least 3 sequence motifs ATTA per 100 nucleotides over a stretch of at most 200 nucleotides.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "polynucleotide", as used herein, refers to a linear or circular nucleic acid molecule. The term encompasses single as well as partially or completely double-stranded polynucleotides. Preferably, the polynucleotide is RNA or is DNA, including cDNA. Moreover, comprised are also chemically modified polynucleotides including naturally occurring modified polynucleotides such as glycosylated or methylated polynucleotides or artificially modified derivatives such as biotinylated polynucleotides. The polynucleotide of the present invention shall be provided, preferably, either as an isolated polynucleotide (i.e. isolated from its natural context) or in genetically modified form. The polynucleotide of the invention comprises at least one promoter active in a host cell and an S/MAR element; moreover, the polynucleotide has the biological activity of replicating episomally in a host cell, all as specified herein below. Preferably, the polynucleotide has a length of at most 1 Mb, more preferably at most 500 kb, even more preferably at most 200 kb, most preferably at most 100 kb. Preferably, the polynucleotide is a non-naturally occurring polynucleotide; thus, preferably, the nucleotide is an artificial polynucleotide. Also preferably, the polynucleotide is a chimeric polynucleotide; more preferably, the polynucleotide comprises at least one nucleic acid sequence heterologous to the remaining nucleic acid sequences it comprises.

As used herein, the term polynucleotide, preferably, includes variants of the specifically indicated polynucleotides. More preferably, the term polynucleotide relates to the specific polynucleotides indicated. The term "polynucleotide variant", as used herein, relates to a variant of a polynucleotide related to herein comprising a nucleic acid sequence characterized in that the sequence can be derived from the aforementioned specific nucleic acid sequence by at least one nucleotide substitution, addition and/or deletion, wherein the polynucleotide variant shall have the biological activity or activities as specified for the specific polynucleotide. Thus, it is to be understood that a polynucleotide variant as referred to in accordance with the present invention shall have a nucleic acid sequence which differs due to at least one nucleotide substitution, deletion and/or addition. Preferably, said polynucleotide variant comprises an ortholog, a paralog or another homolog of the specific polynucleotide or of a functional subsequence thereof, e.g. of an S/MAR element. Also preferably, said polynucleotide variant comprises a naturally occurring allele of the specific polynucleotide or of a functional subsequence thereof. Polynucleotide variants also encompass polynucleotides comprising a nucleic acid sequence which is capable of hybridizing to the aforementioned specific polynucleotides or functional subsequences thereof, preferably, under stringent hybridization conditions. These stringent conditions are known to the skilled worker and can be found in standard textbooks A preferred example for stringent hybridization conditions are hybridization conditions in 6x sodium chloride/sodium citrate (= SSC) at approximately 45°C, followed by one or more wash steps in 0.2x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of O.lx to 5x SSC (pH 7.2). If organic solvent is present in the abovementioned buffer, for example 50% formamide, the temperature under standard conditions is approximately 42°C. The hybridization conditions for DNA:DNA hybrids are preferably for example 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G+C content of 50% in the absence of formamide; accordingly, other conditions more suitable for low-G+C DNA, which are in principle known to the skilled person, may be found to be more appropriate by the skilled person. The skilled worker knows how to determine the hybridization conditions required by referring to standard textbooks. Alternatively, polynucleotide variants are obtainable by PCR-based techniques such as mixed oligonucleotide primer- based amplification of DNA, i.e. using degenerated primers against conserved domains of a polypeptide of the present invention. Conserved domains of a polypeptide may be identified by a sequence comparison of the nucleic acid sequence of the polynucleotide or the amino acid sequence of the polypeptide of the present invention with sequences of other organisms. As a template, DNA or cDNA from bacteria, fungi, plants or, preferably, from animals may be used. Further, variants include polynucleotides comprising nucleic acid sequences which are at least 70%, at least 75%, at least 80%, at least 85%, at least 90%, at least 95%, at least 98% or at least 99% identical to the specifically indicated nucleic acid sequences or functional subsequences thereof. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences specifically indicated. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981))), are preferably used. Preferably, said programs are used with their standard parameters. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the specifically indicated nucleic acid sequences, said polynucleotide retaining the indicated activity or activities, is also encompassed as a variant polynucleotide of the present invention. A fragment as meant herein, preferably, comprises at least 200, preferably at least 300, more preferably at least 400 consecutive nucleotides of any one of the specific nucleic acid sequences; or encodes an amino acid sequence comprising at least 100, preferably at least 200, more preferably at least 300 consecutive amino acids of any one of the specific amino acid sequences and still having the indicated activity.

The polynucleotides of the present invention either consist, essentially consist of, or comprise the aforementioned nucleic acid sequences. Thus, they may contain further nucleic acid sequences as well. Specifically, the polynucleotides of the present invention may encode e.g. fusion proteins or selectable markers. Such fusion proteins may comprise as additional part polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and are described elsewhere herein.

Also preferably, the polynucleotide comprises at least one cargo sequence. The term "cargo sequence", as used herein, relates to a nucleic acid sequence of interest of being transferred into and stably maintained in a host cell. Preferably, the cargo sequence is a nucleic acid sequence encoding a polynucleotide, e.g. an RNA, and/or a polypeptide of interest. Preferably, the polypeptide of interest is a therapeutic polypeptide, more preferably a T Cell Receptor (TCR), more preferably a human or chimeric T Cell receptor, a Chimeric Antigen Receptor (CAR), preferably MARTI TCR, or a polypeptide lacking in cells affected with a genetic disease as specified elsewhere herein. Thus, e.g. preferably, the polynucleotide comprises at least one cargo sequence encoding a polypeptide providing phenylalanine-hydroxylase activity (EC 1.14.16.1) for treatment of phenylketonuria.

Preferably, the sequence encoding a selectable marker and the cargo sequence are intervened by a sequence enabling expression of two (or more) polypeptides in a eukaryotic cell from one mRNA, e.g. an internal ribosomal entry sequence (IRES) or, more preferably, a self-cleaving peptide sequence such as, most preferably, a peptide 2A (P2A) sequence from porcine teschovirus-1. Appropriate sequences are known in the art, e.g. from Kim et al. (2011) PLoS ONE 6(4): e18556.

Preferably, the polynucleotide is a DNA. Preferably, the polynucleotide comprises further expression control sequences allowing expression of genes in prokaryotic and/or eukaryotic, preferably in eukaryotic host cells or isolated fractions thereof. Expression of said polynucleotide comprises transcription of the polynucleotide, preferably into a translatable mRNA. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Examples for regulatory elements permitting expression in eukaryotic host cells are the AOX1 or GAL1 promoter in yeast or the SMVP-, U6-, H1-, 7SK-, CMV- EFS-, SV40-, or RSV-promoter (Rous sarcoma virus), CMV-enhancer, SV40-enhancer or a globin intron in mammalian and other animal cells. Moreover, inducible or cell type-specific expression control sequences may be comprised in a polynucleotide of the present invention. Inducible expression control sequences may comprise tet or lac operator sequences or sequences inducible by heat shock or other environmental factors. Suitable expression control sequences are well known in the art. Besides elements which are responsible for the initiation of transcription, such regulatory elements may also comprise transcription termination signals, such as the SV40-poly-A site or the tk-poly-A site, downstream of the polynucleotide.

The term "host cell", as used herein, relates to any cell capable of receiving and stably replicating the polynucleotide. Preferably, the host cell is a eukaryotic cell, preferably a plant or yeast cell, e.g. a cell of a strain of baker's yeast, or is an animal cell. More preferably, the host cell is an insect cell or a mammalian cell, in particular a mouse or rat cell. Even more preferably, the host cell is a mammalian cell, most preferably is a human cell. Preferably, the host cell is a CD34+ Progenitor Cell; a CD61+ Thrombocyte; a CD19+ B-Lymphocyte; a CD14+ Monocyte; a CD15+ Granulocyte; a CD3+ Cytotoxic T-Lymphocyte, preferably also positive for CD8 and CD45; a CD3+ Helper T-Lymphocyte, preferably also positive for CD4 and CD45; a CD3+ activated T-Lymphocyte, preferably also positive for CD25 and CD45, a Tumor infiltrating Lymphocyte, or a Natural Killer (NK) cell. As will be understood by the skilled person, the polynucleotide may in addition have sequences permitting replication in a bacterial cell, in particular a bacterial origin of replication. Preferably, the bacterial cell is a cell of a laboratory strain of bacteria, more preferably an Escherichia coli cell.

The term "promoter" is, in principle, known to the skilled person as a genetic element directing, optionally in concert with further regulatory elements, the level of transcription of a given gene. A promoter may be constitutive, i.e. providing a constant level of transcription essentially independent of a host cell's state, or may be regulated, i.e. provide levels of transcription in dependence of a host cell's state. Moreover, a promoter may be cell type and/or tissue specific, i.e. provide a detectable level of transcription only in a few or only one cell type. Preferably, the promoter according to the present invention is active in the host cell as specified herein above. As will be understood by the skilled person, the selection of promoter may depend on the type of host cell intended for targeting; suitable promoters for specific cell types as well as constitutive promoters are known in the art. Preferably, the promoter is a eukaryotic promoter, more preferably a constitutive eukaryotic promoter, even more a strong eukaryotic promoter. Preferably, the promoter is an EF1alpha (elongation factor 1 alpha) promoter, an UbiC (ubiquitin C) promoter, a ROSA 26 promoter, a PGK (phosphoglycerate kinase) promoter, and/or a CAG (chicken alpha-actin) promoter, more preferably is an EF1alpha promoter. Also preferably, the promoter is a cell- and/or tissue-specific eukaryotic promoter. As used herein, the term "promoter" is used for the promoter as specified above, whereas any other promoter potentially present on the polynucleotide in addition is referred to as "secondary promoter". Thus, preferably, the promoter is a promoter directing transcription into the S/MAR sequence in a host cell; also preferably, a promoter not directing transcription into the S/MAR sequence of the polynucleotide, e.g. for being a prokaryotic promoter, for being transcriptionally insulated from the S/MAR sequence, and/or for being a promoter directing transcription away from the S/MAR sequence, is a secondary promoter. Preferably, the promoter comprises less than 1000, more preferably less than 250, even more preferably less than 100, most preferably less than 20 contiguous base pairs corresponding to an Apolipoprotein B promoter; thus, preferably, the polynucleotide does not comprise a human Apolipoprotein B promoter, more preferably does not comprise an Apolipoprotein B promoter.

Preferably, the S/MAR sequence is located immediately downstream of the promoter and, if present, of the selectable marker gene as specified herein below. Preferably, being located "immediately downstream" is lacking an intervening transcription termination signal, more preferably is lacking an intervening gene. Thus, preferably, transcripts initiated at the promoter and, if encoded, including the detectable marker sequence preferably comprise a transcribed S/MAR sequence, more preferably comprise the complete S/MAR sequence comprised in the polynucleotide; as will be understood by the skilled person in view of the description elsewhere herein, the polynucleotide may further include splicing sites mediating excision of the S/MAR sequence from the primary transcript; thus, more preferably, preferably, at least primary transcripts initiated at the promoter and, if encoded, including the detectable marker sequence preferably comprise a transcribed S/MAR sequence, more preferably comprise the complete S/MAR sequence comprised in the polynucleotide. Also preferably, the term "immediately downstream" includes a polynucleotide in which the promoter and the S/MAR sequence are separated by elongated nucleic acid sequences, provided a transcription termination signal is not intervening the promoter and the S/MAR. Preferably, the sequence intervening the promoter or, if present, the stop codon of the selectable marker gene and the S/MAR sequence has a length of at most 2 kb, more preferably at most 0.5 kb, even more preferably at most 0.2 kb, still more preferably at most 0.1 kb, most preferably at most 50 bp.

The term "S/MAR element", also known under the designation "scaffold/matrix attachment region", is, in principle, known to the skilled person to relate to a DNA sequence mediating attachment of the nuclear matrix of a eukaryotic cell to said DNA. S/MAR sequences typically are derived from sequences in the DNA of eukaryotic chromosomes. A variety of S/MAR sequences is available, and sequences are available from public databases, e.g. as described in Liebich et al. (2002), Nucleic Acids Res. 30, 312-374. According to the present invention, the nucleic acid sequence of said S/MAR element (hitherto referred to as S/MAR sequence) comprises at least 3 sequence motifs ATTA per 100 nucleotides over a stretch of at most 200 nucleotides. Thus, the motif comprised in the S/MAR sequence comprises a multitude of the four-nucleotide motif 5'-ATTA-3'. Preferably, the S/MAR sequence has a length of at least 200 nucleotide, more preferably at least 300 nucleotides, even more preferably at least 400 nucleotides, most preferably at least 500 nucleotides. Preferably, the S/MAR sequence has a length of at most 3 kb, more preferably at most 2 kb, even more preferably at most 1.5 kb, still more preferably at most 1 kb, most preferably at most 0.9 kb. Thus, preferably, the S/MAR sequence has a length of from 0.2 kb to 3 kb, more preferably of from 0.3 kb to 2 kb, even more preferably of from 0.4 kb to 1.5 kb, most preferably of from 0.5 kb to 1 kb. As will be understood, the indication "comprises n sequence motifs per 100 nucleotides" relates to the average number of said sequence motifs calculated per 100 base pairs of sequence and, accordingly, may be a fraction number. E.g. the number of ATTA sequence motifs per 100 base pairs in SEQ ID NO:6 is 34 / 525 base pairs * 100 base pairs = 6.5. Preferably, the number of sequence motifs per 100 base pairs is determined over the whole length of the S/MAR sequence; in case of doubt, e.g. where a boundary of the S/MAR sequence cannot be determined, the number of sequence motifs per 100 base pairs of a polynucleotide, preferably, is the highest number determinable for any window of 200 bp within said polynucleotide, more preferably is the highest number determinable for any window of 500 bp within said polynucleotide. Preferably, the S/MAR sequence comprises at least 4 sequence motifs ATTA per 100 nucleotides over a stretch of at most 200 nucleotides, more preferably at least 5 sequence motifs ATTA per 100 nucleotides over a stretch of at most 200 nucleotides, still more preferably at least 6 sequence motifs ATTA per 100 nucleotides over a stretch of at most 200 nucleotides. Also preferably, the S/MAR sequence comprises at least 3 sequence motifs ATTA per 100 nucleotides over a stretch of at most 400 nucleotides, more preferably at least 4 sequence motifs ATTA per 100 nucleotides over a stretch of at most 400 nucleotides, even more preferably at least 5 sequence motifs ATTA per 100 nucleotides over a stretch of at most 400 nucleotides, most preferably at least 6 sequence motifs ATTA per 100 nucleotides over a stretch of at most 400 nucleotides. Also preferably, the S/MAR sequence comprises at least 3 sequence motifs ATTA per 100 nucleotides over a stretch of at most 500 nucleotides, more preferably at least 4 sequence motifs ATTA per 100 nucleotides over a stretch of at most 500 nucleotides, still more preferably at least 5 sequence motifs ATTA per 100 nucleotides over a stretch of at most 500 nucleotides, most preferably at least 6 sequence motifs ATTA per 100 nucleotides over a stretch of at most 500 nucleotides. Thus, preferably, the S/MAR sequence comprises at least 10 sequence motifs ATTA over a sequence of 500 nucleotides, more preferably at least 20 sequence motifs ATTA over a sequence of 500 nucleotides, still more preferably at least 30 sequence motifs ATTA over a sequence of 500 nucleotides. Preferably, at least 80%, more preferably at least 90%, most preferably at least 95% of the ATTA motifs in the S/MAR sequence are separated by of from 9 to 13, preferably by 10 to 12, most preferably by 11 base pairs, respectively.

The S/MAR element comprises additional sequence motifs within the sequence comprising the ATTA motifs described herein above. Preferably, the sequence stretch of said S/MAR element comprising said ATTA sequence motifs further comprises at least one sequence motif ATTTA (SEQ ID NO:2), preferably at least 2 sequence motifs ATTTA, more preferably at least 4 sequence motifs ATTTA, most preferably at least 8 sequence motifs ATTTA. The sequence stretch of said S/MAR element comprising said ATTA sequence motifs and, optionally, said ATTTA motif(s), further comprises at least one motif TAAATATTTTA (SEQ ID NO:3); preferably said sequence stretch comprises at least two, more preferably at least four, most preferably at least six palindromic motifs, preferably motifs TAAATATTTTA (SEQ ID NO:3). Preferably, said motifs TAAATATTTTA are contiguous with at least one motif ATTA on the 5' end and/or the 3' end. Also preferably, the sequence stretch of the S/MAR element comprising said ATTA sequence motifs comprises at least one, preferably at least two, more preferably at least three, even more preferably at least four, most preferably at least five sequence motifs ATTATAAATATTTTAATTA (SEQ ID NO:4), more preferably sequence motifs ATTTAATTATAAATATTTTAATTA (SEQ ID NO:5).

Also preferably, the S/MAR sequence has a low G+C content. The skilled person knows how to calculate the C+G content of a known sequence by counting all guanine and cytidine bases in the sequence and dividing the cumulated result by the number of nucleotides in the sequence. Preferably, the sequence stretch of the S/MAR element comprising said sequence motifs ATTA has a G+C content of at most 30%, more preferably at most 20%, still more preferably at most 15%, even more preferably at most 10%, most preferably at most 5%. Preferably, in cases where the boundary of an S/MAR element cannot be determined, the sequence used for calculation of the G+C content is the same used for calculating the number of ATTA motifs per 100 base pairs, as specified herein above. Also preferably, the S/MAR sequence has a low number of CG dinucleotides. Preferably, the sequence stretch of said S/MAR element comprising said sequence motifs comprises at most 6 sequence motifs CG, more preferably at most 4, even more preferably at most 2, most preferably does not comprise a sequence motif CG.

Preferably, the S/MAR sequence comprises an S/MAR sequence of an Apolipoprotein B gene, preferably a human Apolipoprotein B gene, more preferably a 3' S/MAR sequence of a human Apolipoprotein B gene. More preferably, the S/MAR sequence comprises a variant of a human Apolipoprotein B gene, more preferably of a 3' S/MAR sequence of a human Apolipoprotein B gene. Thus, preferably, the S/MAR sequence comprises a sequence at least 70% identical to the sequence of SEQ ID NO:6, preferably of SEQ ID NO:7 or 8. More preferably, the S/MAR sequence comprises the nucleic acid sequence of SEQ ID NO:6, preferably of SEQ ID NO:7, more preferably SEQ ID NO:8.

Preferably, the polynucleotide comprises a poly-A signal downstream of the S/MAR element: More preferably, the polynucleotide comprises a poly-A signal and a transcription termination signal downstream of the S/MAR element. Also preferably, the S/MAR element is flanked by a splice donor and a splice acceptor; thus, preferably, the S/MAR sequence preferably is spliced out of the transcript encoding the selectable marker after transcription. Also preferably, the polynucleotide further comprises a (secondary) bacterial origin of replication as specified herein above and/or a bacterial selectable marker gene. Preferably, the bacterial origin of replication and the promoter driving expression of the bacterial selectable marker gene are prokaryote-specific, i.e., more preferably, are non-functional in a host cell. Also preferably, the bacterial origin of replication and/or bacterial selectable marker gene, preferably all elements active in a prokaryotic cell comprised in the polynucleotide, is/are insulated from the residual sequences comprised in the polynucleotide by the presence of at least one insulation element, more preferably by being flanked by insulation elements. preferably, the bacterial origin of replication and/or bacterial selectable marker gene, preferably all elements active in a prokaryotic cell, is/are insulated from the residual sequences comprised in the polynucleotide by the presence of at least one insulating element at the 5' end and of at least one insulating element at the 3' end. More preferably, the bacterial origin of replication and/or bacterial selectable marker gene, preferably all elements active in a prokaryotic cell comprised in the polynucleotide, is/are insulated from the promoter by the presence of at least one insulation element, more preferably by being flanked by insulation elements. Preferably, said insulation element(s) is(are) an anti-repressive element40 element (SEQ ID NO:11) or a variant thereof and/or an S/MAR element.

Thus, preferably, the polynucleotide comprises the sequence of SEQ ID NO:7 or 8 or of a sequence at least 70% identical to the sequence of SEQ ID NO:7 or 8; preferably of SEQ ID NO:12 or of a sequence at least 70% identical to the sequence of SEQ ID NO: 12, more preferably of SEQ ID NO:13 or of a sequence at least 70% identical to the sequence of SEQ ID NO:13, most preferably of SEQ ID NO:14 or of a sequence at least 70% identical to the sequence of SEQ ID NO:14. Preferably, the polynucleotide comprises the sequence of SEQ ID NO:14 with the nucleic acid sequence encoding GFP replaced by a nucleic acid sequence encoding a different polypeptide, preferably a therapeutic polypeptide, more preferably human T Cell Receptor (TCR), Chimeric Antigen Receptor (CAR), preferably MARTI TCR.

Preferably, the polynucleotide further comprises a coding sequence encoding a selectable marker polypeptide, said selectable marker sequence intervening the promoter of the polynucleotide and the S/MAR element of the polynucleotide, preferably wherein said promoter and said selectable marker sequence together constitute a selectable marker gene. As used herein, the term "selectable marker sequence" is used as a shorthand for the expression "coding sequence encoding a selectable marker polypeptide". The term "selectable marker" is in principle understood by the skilled person and relates to a nucleic acid sequence conferring, when expressed in a host cell, resistance to at least one condition mediating selective pressure to a host cell when applied thereto. Selectable markers are known in the art for prokaryotic and for eukaryotic cells. Preferably, the selectable marker is a selectable marker of an eukaryotic cell. Preferably, the selectable marker is a selectable marker polypeptide, more preferably a selectable marker polypeptide having transporter and/or enzymatic activity removing a selective compound from a hot cell or modifying said selective compound to make it inactive. Preferably, the selectable marker gene further encodes at least one intron, preferably upstream of the sequence encoding the selectable marker polypeptide. Preferably, the selectable marker is a marker mediating resistance to puromycin, to blasticidin, neomycin, and/or to zeocin, more preferably to puromycin. Thus, preferably, the promoter and the selectable marker together constitute a puromycin resistance gene, a blasticidin resistance gene, a neomycin resistance gene, or a zeocin resistance gene, more preferably a puromycin resistance gene. Preferably, the selectable marker gene is devoid of a poly-A signal and of transcription termination signal(s).

Preferably the selectable marker is the puromycin acetyltransferase (Genbank Acc No. KX548903.1 (SEQ ID NO:9), encoded by nucleotides 535 to 1134 of Genbank Acc No. KX548903.1 (SEQ ID NO:10)). Thus, the selectable marker gene, preferably, comprises a nucleic acid sequence which a) causes expression of a puromycin resistance polypeptide comprising the sequence of SEQ ID NO:9; b) causes expression of a puromycin resistance polypeptide comprising a sequence at least 70% identical to the sequence of SEQ ID NO:9; c) comprises the sequence of SEQ ID NO:10; d) comprises a sequence at least 70% identical to the sequence of SEQ ID NO:10, e) comprises a nucleic acid sequence encoding a puromycin resistance polypeptide comprising, preferably consisting of, the sequence of SEQ ID NO:9, and/or f) comprises a nucleic acid sequence encoding a puromycin resistance polypeptide comprising, preferably consisting of, a sequence at least 70% identical to the sequence of SEQ ID NO:9.

As used herein, the term "replicating" relates to the activity of the polynucleotide to induce production of at least two replicas of said polynucleotide in a host cell during a cell replication cycle. Thus, preferably, replication of a polynucleotide in a host cell is determined by determining the presence of the polynucleotide after a series of cell divisions, in which a non-replicating polynucleotide would have been expected to be diluted out. Preferably, replication is stable replication, i.e. is replication to such an extent that the polynucleotide still is detectable in a host cell population after on average 50 cell divisions, more preferably after on average 100 cell divisions, most preferably after on average 250 cell divisions. Preferably, detection of a polynucleotide in a host cell population is performed by PCR under standard conditions.

The term "episomal" replication is, in principle, known to the skilled person to relate to replication of a polynucleotide without being integrated into the cellular genome, i.e. without becoming covalently attached to the cellular genome. Thus, preferably, episomal replication of a polynucleotide is replication of said polynucleotide as an autonomous replication unit. Preferably, episomal replication is maintenance of the polynucleotide in the host cell in the form of a circularly closed double-stranded DNA molecule. As will be understood by the skilled person, the actual replication of said polynucleotide may involve other forms, e.g. in rolling circle replication. Episomal maintenance of circular DNA preferably is verified by the plasmid rescue procedure known to the skilled person; i.e. preferably, by preparing a lysate of host cells and transforming the DNA comprised therein into appropriate bacterial cells, e.g. E. coli cells; if a suitable number of bacterial colonies obtainable by said method comprises the circular DNA as a plasmid having the same restriction pattern and/or sequence as the original circular DNA, it is, preferably, assumed that the circular DNA was maintained episomally. A further method of verifying episomal maintenance, which is also known to the skilled person, is DNA/DNA blotting ("Southern Blot" method); thus, preferably, total DNA of host cells is prepared and digested with one or more restriction enzyme(s); if in a Southern Blot using the original plasmid as a probe only bands corresponding to the original circular DNA are visible, it is preferably concluded that the plasmid is maintained episomally. More preferably, episomal maintenance is verified as described herein in the Examples.

In accordance, the term "replicating episomally", as used herein, relates to the activity of a polynucleotide to induce production of at least two replicas of said polynucleotide in a host cell during a cell replication cycle while said polynucleotide is present in said cell as an autonomously replicating entity; and stable episomal replication is episomal replication to such an extent that the polynucleotide is still detectable in the host cell after at least 50 cell divisions, preferably after at least 100 cell divisions, more preferably, after at least 250 cell divisions, most preferably, after at least 500 cell divisions. Preferably, the aforesaid number of cell divisions is the average number of cell divisions for a population of cells.

The polynucleotide of the present invention preferably is devoid of a of a simian virus 40 (SV40) origin of replication, a bovine papillomavirus (BPV) origin of replication, and an Epstein-Barr virus (EBV) origin of replication, preferably is devoid of a polyomavirus origin of replication, a papillomavirus origin of replication, and a herpesvirus origin of replication; more preferably is devoid of an origin of replication of an eukaryote-infecting virus. More preferably, the vector is devoid of any known eukaryotic origin of replication. However, preferably, the polynucleotide further comprises a prokaryotic, preferably a bacterial origin of replication, in particular an E. coli origin of replication. Preferably, the prokaryotic origin of replication is the only origin of replication comprised in the polynucleotide.

Advantageously, it was found in the work underlying the present invention that by combining an S/MAR element as specified with a promoter reading into said S/MAR element, a polynucleotide is obtained which is highly stable in episomal form in host cells, even in the absence of a dedicated origin of replication. Moreover, it was found that efficacy of establishment of the polynucleotide could be further improved by using a puromycin resistance gene, by ensuring transcription into the S/MAR element through the resistance gene, and by insulating the promoter - S/MAR combination transcriptionally from other promoters potentially present in the polynucleotide.

The definitions made above apply mutatis mutandis to the following. Additional definitions and explanations made further below also apply for all embodiments described in this specification mutatis mutandis.

The present invention further relates to a composition according to claim 9; also disclosed is a composition comprising a polynucleotide according to the present invention.

The term "composition", as used herein, as used herein, relates to a composition of matter comprising the compounds as specified and optionally one or more acceptable carrier. Preferably, the composition is a pharmaceutically acceptable composition; thus, preferably, the carrier is a pharmaceutically acceptable carrier. The compounds of the present invention can be formulated as, preferably pharmaceutically acceptable, salts. Preferred salts comprise acetate, methylester, HCl, sulfate, chloride and the like.

The carrier(s) must be acceptable in the sense of being compatible with the other ingredients of the formulation and being not deleterious to the recipient thereof. A carrier employed may be, for example, either a solid, a gel or a liquid. Exemplary of solid pharmaceutical carriers are lactose, terra alba, sucrose, talc, gelatin, agar, pectin, acacia, magnesium stearate, stearic acid and the like. Exemplary of liquid carriers are phosphate-buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The diluent(s) is/are selected so as not to affect the biological activity of the compounds in the composition. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

Preferably, the composition mediates entry of the polynucleotide into a host cell. Thus, preferably the composition comprises at least one transfection agent. The selection of an appropriate transfection agent may depend on the target host cell, as well as the specific application envisaged. Transfection agents, appropriate transfection conditions, as well as selection criteria therefor are well-known in the art. Also preferably, the composition comprises virus-like particles. Thus, preferably, the polynucleotide is packaged into virus-like particles, i.e. preferably, the polynucleotide is comprised in the virus-like particles.

Pharmaceutical compositions are, preferably, administered topically or systemically. Suitable routes of administration conventionally used for drug administration are oral, intravenous, or parenteral administration as well as inhalation. However, depending on the nature and mode of action of a compound, the pharmaceutical compositions may be administered by other routes as well. For example, polynucleotide compounds may be administered in a gene therapy approach by using viral vectors or viruses or liposomes, as specified herein above. Moreover, the compounds can be administered in combination with other drugs either in a common pharmaceutical composition or as separated pharmaceutical compositions wherein said separated pharmaceutical compositions may be provided in form of a kit of parts. The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

A therapeutically effective dose of a pharmaceutical composition refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass. In case a viral vector, in particular adeno-associated viral vector is administered, preferred doses are from 5 x 10¹¹ to 2 x 10¹³ viral particles or viral genomes / kg body weight; as will be understood, these exemplary doses may be modified depending, in addition to the factors described above, on additional factors like type of virus, target organ, and the like.

The pharmaceutical compositions and formulations referred to herein are administered at least once in order to treat or ameliorate or prevent a disease or condition recited in this specification. However, the said pharmaceutical compositions may be administered more than one time, for example from one to four times daily up to a non-limited number of days.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescribers or users instructions in order to anticipate dose adjustments depending on the considered recipient.

The present invention also relates to a polynucleotide according to the present invention, a composition according to the present invention, and/or a host cell according to the present invention, for use in medicine according to claim 11. The present invention further relates to a polynucleotide according to the present invention, a composition according to the present invention, and/or a host cell according to the present invention, for use in treating genetic disease according to claim 11.

The term "genetic disease", as used herein, relates to a disease causally linked to one or more modifications, preferably mutations, in the genome of an individual. Thus, preferably, the genetic disease is causally linked to one or more epigenetic changes, more preferably is causally linked to one or more genetic mutations. As will be understood, symptoms of a genetic disease often are caused by expression of a mutated gene and/or lack of expression of a gene providing normal function of the gene product in one or more specific tissue(s) and/or cell type(s). Thus, it may be preferable to treat genetic disease only in those cells in which the mutation contributes to disease. Preferably, the genetic disease is a monogenic disease, i.e. is caused by a genetic alteration in one gene. More preferably, the genetic disease is a monogenic recessive disease, i.e. is caused by genetic alterations in both alleles of a gene; thus, preferably, the amelioration of symptoms is expected by provision of at least one unaltered copy of the affected gene. Most preferably, the genetic disease is phenylketonuria, alkaptonuria, Leber's Congenital Amaurosis, Choroideremia, or Stargardt disease.

The present invention also relates to a kit according to claim 12; also disclosed is a kit comprising a polynucleotide according to the present invention and a compound mediating cell entry.

The term "kit", as used herein, refers to a collection of the aforementioned compounds, means or reagents of the present invention which may or may not be packaged together. The components of the kit may be comprised by separate vials (i.e. as a kit of separate parts) or provided in a single vial. Moreover, it is to be understood that the kit of the present invention, preferably, is to be used for practicing the methods referred to herein above. It is, preferably, envisaged that all components are provided in a ready-to-use manner for practicing the methods referred to above. Further, the kit, preferably, contains instructions for carrying out said methods. The instructions can be provided by a user's manual in paper or electronic form. In addition, the manual may comprise instructions for interpreting the results obtained when carrying out the aforementioned methods using the kit of the present invention. As will be understood from the above, the description of the kit comprising polynucleotides, preferably, relates to a kit comprising corresponding vectors mutatis mutandis.

Preferably, the kit further comprises at least one compound mediating cell entry for the polynucleotide it comprises, the term "compound mediating cell entry" relating to any means suitable to cause a polynucleotide of the kit to enter the interior of a host cell, preferably a host cell. Suitable compound mediating cell entry (delivery means) are known in the art and include in particular transfection means, packaging compositions, and the like. Preferably, the polynucleotide of the present invention is pre-packaged in a delivery means, e.g. in viral particles, more preferably in replication-defective viral particles, most preferably in virus-like particles (VLPs). The skilled person is aware of delivery means providing different specificities for cellular receptors, such that delivery means appropriate for a given target host cell may be selected.

The present invention further relates to a device according to claim 13; also disclosed is a device comprising a polynucleotide according to the present invention, a composition according to the present invention, and/or a host cell according to the present invention.

The term "device", as used herein relates to a system of means comprising at least the means operatively linked to each other as to allow administration of the compound or of the composition of the present invention. Preferred means for administering polynucleotides, compositions, host cells are well known in the art. How to link the means in an operating manner will depend on the type of means included into the device and on the kind of administration envisaged. Preferably, the means are comprised by a single device in such a case. Said device may accordingly include a delivery unit for the administration of the compound or composition and a storage unit for storing said compound or composition until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. In a preferred embodiment, the device is a syringe, more preferably with a needle, comprising the compound or composition of the invention. In another preferred embodiment, the device is an intravenous infusion (IV) equipment comprising the compound or composition. In another preferred embodiment, the device is an endoscopic device comprising the compound or medicament for flushing a site of administration, or further comprising a needle for topical application of the compound or composition, e.g. to a tumor. In still another preferred embodiment the device is an inhaler comprising the compound of the present invention, wherein, more preferably, said compound is formulated for administration as an aerosol.

The present application also relates to a method for stably transfecting a host cell according to claim 14; also disclosed is a method for stably transfecting a host cell, comprising
a) contacting said host cell with a polynucleotide according to the present invention, a composition according to the present invention, and/or a host cell according to the present invention, and,
b) thereby, stably transfecting a host cell.

The method for stably transfecting a host cell of the present invention, preferably, is an in vitro method. Moreover, it may comprise steps in addition to those explicitly mentioned above. For example, further steps may relate, e.g., to providing a host cell or a sample comprising the same for step a), and/or applying selective pressure to the host cells contacted. Moreover, one or more of said steps may be performed by automated equipment.

The term stably transfecting a host cell is understood by the skilled person to relate to introducing a polynucleotide, preferably a heterologous polynucleotide into a cell such that the polynucleotide is stable replicated by the host cell as specified herein above. Preferably, stable transfection comprises stable episomal replication of the polynucleotide. Preferably, stable transfecting comprises, after contacting, applying selective pressure to the host cell to select for the presence of a selectable marker. The selective pressure is applied after contacting, optionally excluding a first time frame allowing the polynucleotide to establish within the host cell; the duration of said first time frame allowing the polynucleotide to establish within the host cell will depend mostly on the type of host cell contacted and on the kind of selectable marker used; preferably, the duration of said first time frame allowing the polynucleotide to establish within the host cell is of from 1 h to 48 h, more preferably of from 2 h to 24, most preferably of from 3 h to 16 h. However, the duration of said first time frame allowing the polynucleotide to establish within the host cell may also be zero, i.e. selective pressure may be applied immediately after contacting or even during contacting. Selective pressure may be applied continuously, i.e. at essentially all time points after the first time frame allowing the polynucleotide to establish within the host cell, more preferably to prevent host cells not comprising the polynucleotide from proliferating; or it may be applied transiently, more preferably to remove cells not having received the polynucleotide. Preferably, transient application of selective pressure is used in cases where cells are transferred back into an organism after said contacting. It is, however, also envisaged that no selective pressure is applied, in particular in cases where it is known that the efficiency of transfer of the polynucleotide into target host cells is sufficiently high and/or where a pure population of transgenic host cells is not of major importance.

The term "contacting", as used in the context of the methods of the present invention, is understood by the skilled person. Preferably, the term relates to bringing at least one polynucleotide, vector, and/or host cell of the present invention in physical contact with a host cell, e.g. allowing the host cell and the compound(s) to interact. Preferably, contacting includes delivery of at least one polynucleotide of the present invention into the interior of a host cell, preferably via a delivery means as specified above.

The present disclosure also relates to a method for treating genetic disease in a subject, comprising
a) contacting said subject with a polynucleotide according to the present invention, a composition according to the present invention, and/or a host cell according to the present invention, and,
b) thereby, treating genetic disease in said subject.

The method for treating genetic disease of the present invention, preferably, is an in vivo method. Moreover, it may comprise steps in addition to those explicitly mentioned above. For example, further steps may relate, e.g., to providing a host cell or a sample comprising the same for step a), and/or re-administering said sample or host cell into the subject. Thus, the method for treating genetic disease, comprise the steps of the method for stably transfecting a host cell as specified above. Moreover, one or more of said steps may be performed by automated equipment.

Further, the present invention relates to a use according to claim 15; also disclosed is a use of a polynucleotide of the present invention for stably genetically modifying a host cell.

Also, the present invention relates to a use of a polynucleotide according to the present invention, a composition according to the present invention, and/or a host cell according to the present invention, for the manufacture of a medicament. And to a use of a polynucleotide according to the present invention, a composition according to the present invention, and/or a host cell according to the present invention, for the manufacture of a medicament for treating genetic disease, preferably monogenic disease, more preferably monogenic recessive disease, most preferably phenylketonuria, alkaptonuria, Leber's Congenital Amaurosis, Choroideremia, or Stargardt disease.

Also, the present invention relates to a use of a polynucleotide according to the present invention, a composition according to the present invention, and/or a host cell according to the present invention, for the genetic modification of a primary cell, preferably a primary dermal fibroblast, for the generation of an Induced Pluripotent Stem Cells (IPSCs). Preferably, said primary cell is a mouse or a human primary cell.

The term "primary cell" is understood by the skilled person as opposed to a cell of a cultured cell line; thus, preferably, a primary cell is a cell derived from a living organism and having been cultured for at most 20 passages, more preferably at most 15 passages, even more preferably at most 10 passages, still more preferably at most 5 passages. Most preferably, primary cells are cells derived directly from tissue of a living being, preferably a mouse or a human.

The term "stem cell" is also understood by the skilled person to relate to an un- or low-differentiated cell with the potential for differentiation into at least two cell types, preferably at least five cell types, more preferably at least one complete cell lineage. Preferably, the stem cell is a totipotent stem cell, more preferably a pluripotent stem cell. The term "Induced Pluripotent Stem Cell" or "IPSC" relates to a pluripotent stem cell derived from a differentiated cell, preferably a differentiated primary cell. Methods of generating IPSCs are known in the art and include, preferably, expression of four transcription factors in the cell (e.g from Takahashi et al. (2006), Cell. 126 (4):663).

The present disclosure also relates to a use of a polynucleotide according to the present invention, a composition according to the present invention, and/or a host cell according to the present invention, for the genetic modification of embryonic stem cells. The present disclosure also relates to a use of a polynucleotide according to the present invention, a composition according to the present invention, and/or a host cell according to the present invention, for the manufacture of a medicament for treating genetic disease, preferably monogenic disease, more preferably monogenic recessive disease, most preferably phenylketonuria, alkaptonuria, Leber's Congenital Amaurosis, Choroideremia, or Stargardt disease, wherein said medicament comprises host cells comprising a polynucleotide of the present invention.

The present disclosure also relates to a use of a polynucleotide according to the present invention, a composition according to the present invention, and/or a host cell according to the present invention, for the genetic modification of stem cells for generating a non human transgenic animal. The present disclosure further relates to a use of a polynucleotide according to the present invention, a composition according to the present invention, and/or a host cell according to the present invention, for the production of a non human transgenic animal.

The term "transgenic animal" as used herein, relates to an non human animal comprising at least one heterologous polynucleotide, preferably introduced into said animal by methods of genetic engineering. Preferably, the transgenic animal comprises at least one, more preferably at least 10, still more preferably at least 1000, even more preferably at least 10000 cells comprising at least one polynucleotide according to the present invention.

Also, the present disclosure relates to a use of a polynucleotide according to the present invention, a composition according to the present invention, and/or a host cell according to the present invention, for the genetic modification of single cell non human embryos by pronuclear injection.

As is understood by the skilled person, the term "pronuclear injection" relates to injecting genetic material, preferably a polynucleotide of the present invention, into the nucleus of a fertilized oocyte, preferably to create a non human transgenic animal.

### Figure Legends

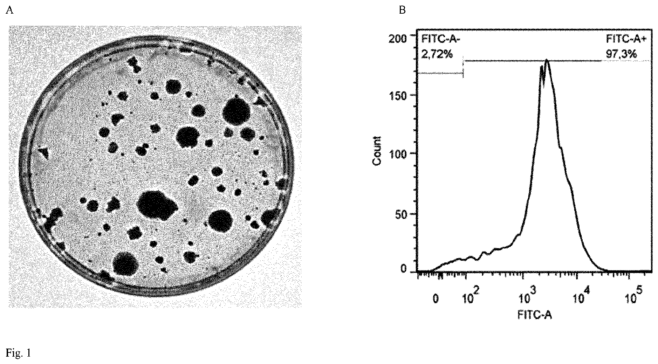

Fig. 1: Efficiency of establishment and analysis of the genetically modified cell population: A) A cell culture plate with Crystal Violet stained colonies having formed after 4 weeks selection with Puromycin; the efficiency of vector establishment was approximately 40%; b) FACS detection of GFP fluorescence in Puromycin selected cells; fluorescence is very homogenous and the number of non-fluorescing cells is extremely low.

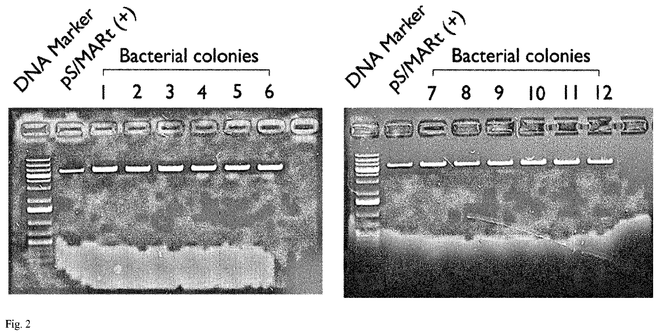

Fig. 2: Result of plasmid rescue of pS/MARt vectors from established cell populations: DNA from bacterial colonies (numbers 1 to 12) obtained in a plasmid rescue experiment were digested with BamHI and were resolved by agarose gel electrophoresis; the lanes labeled with "p/SMART" contain DNA from a colony of bacteria carrying the original plasmid treated the same as above.

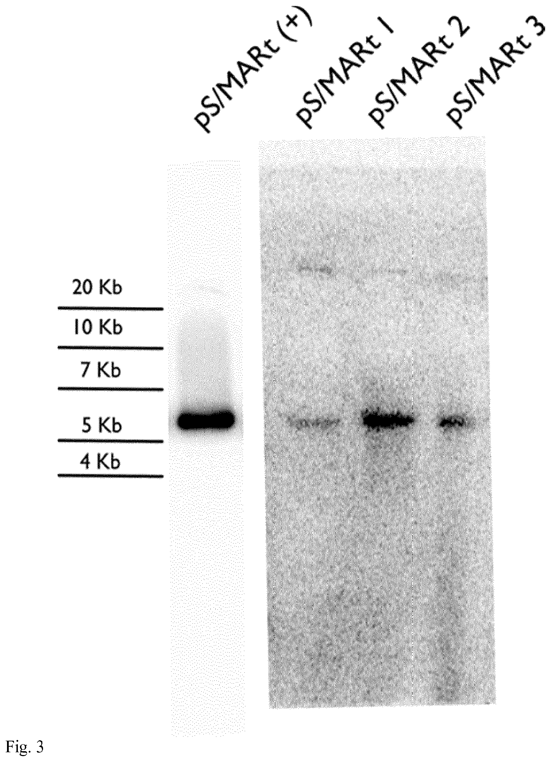

Fig. 3: Southern blot of pSMARt vectors maintained in selected cells: oligonucleotides hybridizing to the GFP gene of pS/MART were used as probes to detect BamHI restricted vector DNA in extracts from host cells (pS/MARt1 to 3); the non-transfected vector was used as a control ("pS/MARt(+)").

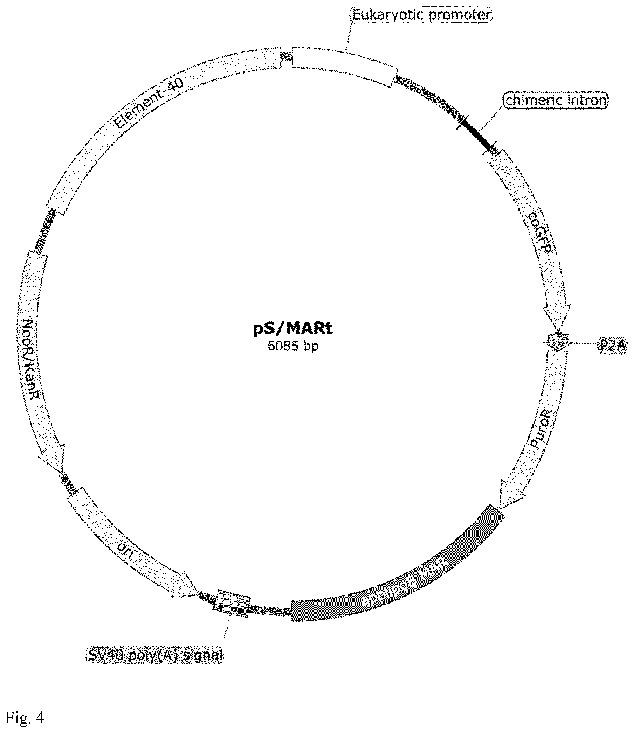

Fig. 4: Vector map of pS/MART; ori: bacterial origin of replication, P2A: sequence encoding the self-cleaving 2A peptide from porcine teschovirus-1, apolipoB MAR: S/MAR sequence from the apolipoprotein B gene.

The following Examples shall merely illustrate the invention. They shall not be construed, whatsoever, to limit the scope of the invention.

### Example 1: Efficiency of establishment and analysis of the genetically modified cell population (Fig. 1)

The efficacy in generating stably expressing cells was evaluated in a colony forming assay using pS/MARt (Fig. 4, SEQ ID NO:14). Upon DNA delivery, cells positive for GFP transgene expression were isolated via FACS sorting (FACS Aria II) and 100 cells were plated into a 6 cm cell culture dish. They were then cultured for 4 weeks in presence of 0.5 µg/ml Puromycin. After 4 weeks the cells were fixed with PFA and the colonies stained with Crystal Violet. The number of colonies is considered as the efficiency of vector establishment, i.e. the number of colonies forming per number of FACS sorted cells plated. The generation of stable cells lines is very effective with over 40 % of transfected cells becoming established (Fig. 1A)). The number of transgene (GFP) expressing cells was estimated by Flow Cytometry. As shown in Fig. 1 b), pS/MARt generates modified populations in which the expression of the transgene is homogenous without significant numbers of negative cells.

### Example 2: Plasmid rescue of pS/MARt vectors from established cell populations (Fig. 2)

An effective method to determine if DNA vectors are maintained episomally with integrity within modified cells is to verify that they can be rescued intact into naive bacteria. To do so, persistently established cell lines modified with the plasmid pS/MARt were cultured in the presence of the antibiotic Puromycin (0.5 µg/ml) for 1 week and expanded for at least 30 days without antibiotic to evaluate vector integrity. Total DNA was prepared from the cells using the Blood&Tissue DNAeasy kit (Qiagen) and transformed into DH10B *E. coli* cells. Bacteria were grown on LB-Agar plates with Kanamycin (50µg/ml). 12 colonies were grown in liquid LB medium with Kanamycin (50 µg/ml) overnight and plasmid DNA was extracted with the MiniprepKit (Qiagen). For the analysis the DNA mini preparations were digested with the restriction enzyme BamHI (Thermo Fisher) for 10 min at 37°C and the restriction pattern was addressed on a 1% agarose gel. As control the DNA used for transfecting the cells at the beginning of the establishment procedure was digested with the same enzyme and run as a reference. These gels illustrate that intact pS/MARt DNA could be isolated form stable modified cell lines and that in every instance the DNA was identical to the originally transfected vectors.

### Example 3: pS/MARt vectors are maintained episomally in modified cells (Fig. 3)

To further demonstrate that the pS/MARt vectors were modifying the mammalian cells as an episome, structure was physically determined by Southern Blot analysis. Hek293T cell populations cultured for at least 30 days after DNA transfection were analyzed. The genomic DNA was extracted with the Blood&Tissue DNAeasy kit (Qiagen) and digested over night at 37°C with the restriction enzyme BamHI (NEB). The total cellular DNA was then separated on a 1% agarose gel and transferred to a nylon membrane. Oligonucleotides corresponding to the vector's GFP gene were used to generate the radioactive probe used to detected the pS/MARt DNA within cellular DNA. The presence in the samples of a single band that has the same size of the control vector demonstrates the episomal status of pS/MARt in the established mammalian cell populations. The absence of smears and/or alternative bands demonstrates that the vectors did not rearrange nor integrate into the cellular genome.

## Claims

1. A polynucleotide comprising at least one promoter and an S/MAR element, wherein said S/MAR element is located downstream of said promoter and wherein the nucleic acid sequence of said S/MAR element (S/MAR sequence) comprises at least 3 sequence motifs ATTA (SEQ ID NO:1) and at least one sequence motif TAAATATTTTA (SEQ ID NO:3) per 100 nucleotides over a stretch of at most 200 nucleotides; preferably comprises at least 20 sequence motifs ATTA over a sequence of 500 nucleotides, wherein said polynucleotide further comprises a coding sequence encoding a selectable marker (selectable marker sequence), said selectable marker sequence intervening said promoter and said S/MAR element, preferably wherein said promoter and said selectable marker sequence together constitute a selectable marker gene.

2. The polynucleotide of claim 1, wherein said selectable marker is a nucleic acid sequence conferring, when expressed in a host cell, resistance to at least one condition mediating selective pressure to a host cell when applied thereto.

3. The polynucleotide of any one of claims 1 or 2, wherein the selectable marker is a selectable marker of a eukaryotic cell.

4. The polynucleotide of any one of claims 1 to 3, wherein said selectable marker gene is a puromycin resistance gene, a blasticidin resistance gene, a neomycin resistance gene, or a zeocin resistance gene, preferably is a puromycin resistance gene.

5. The polynucleotide of any one of claims 1 to 4, wherein the selectable marker gene is devoid of a poly-A signal and of transcription termination signal(s).

6. The polynucleotide of any one of claims 1 to 5, wherein the selectable marker gene comprises a nucleic acid sequence which
a) causes expression of a puromycin resistance polypeptide comprising the sequence of SEQ ID NO:9;
b) causes expression of a puromycin resistance polypeptide comprising a sequence at least 70% identical to the sequence of SEQ ID NO:9;
c) comprises the sequence of SEQ ID NO: 10;
d) comprises a sequence at least 70% identical to the sequence of SEQ ID NO: 10,
e) comprises a nucleic acid sequence encoding a puromycin resistance polypeptide comprising, preferably consisting of, the sequence of SEQ ID NO:9, and/or
f) comprises a nucleic acid sequence encoding a puromycin resistance polypeptide comprising, preferably consisting of, a sequence at least 70% identical to the sequence of SEQ ID NO:9.

7. The polynucleotide of any one of claims 1 to 6, wherein said S/MAR sequence is located immediately downstream of said promoter and of said selectable marker gene, preferably wherein being located immediately downstream is being located in such proximity that transcripts of the selectable marker sequence comprise a transcribed S/MAR sequence.

8. The polynucleotide of any one of claims 1 to 7, wherein said polynucleotide replicates episomally in a host cell, preferably wherein episomal replication is stable episomal replication, preferably in a mammalian cell

9. A composition comprising a polynucleotide according to any one of claims 1 to 8, preferably wherein said composition is a pharmaceutical composition.

10. A host cell comprising the polynucleotide according to any one of claims 1 to 8, preferably wherein said host cell is a CD34+ Progenitor Cell; a CD61+ Thrombocyte; a CD19+ B-Lymphocyte; a CD14+ Monocyte; a CD15+ Granulocyte; a CD3+ Cytotoxic T-Lymphocyte, preferably also positive for CD8 and CD45; a CD3+ Helper T-Lymphocyte, preferably also positive for CD4 and CD45; a CD3+ activated T-Lymphocyte, preferably also positive for CD25 and CD45, a Tumor infiltrating Lymphocyte, or a Natural Killer (NK) cell.

11. A polynucleotide according to any one of claims 1 to 8, a composition according to claim 9, and/or a host cell according to claim 10, for use in medicine, preferably, for use in treating genetic disease, preferably monogenic disease, more preferably monogenic recessive disease, most preferably phenylketonuria, alkaptonuria, Leber's Congenital Amaurosis, Choroideremia, or Stargardt disease.

12. A kit comprising a polynucleotide according to any one of claims 1 to 8 and a compound mediating cell entry.

13. A device comprising a polynucleotide according to any one of claims 1 to 8, a composition according to claim 9, and/or a host cell according to claim 10.

14. A method for stably transfecting a host cell, comprising
a) contacting said host cell with a polynucleotide according to any one of claims 1 to 8, a composition according to claim 9, and/or a host cell according to claim 10, and,
b) thereby, stably transfecting a host cell.

15. Use of a polynucleotide according to any one of claims 1 to 8 for stably genetically modifying a host cell.

## Patentansprüche

1. Polynukleotid, umfassend wenigstens einen Promoter und ein S/MAR-Element, wobei das S/MAR-Element stromabwärts vom Promotor liegt und wobei die Nukleinsäuresequenz des S/MAR-Elements (S/MAR-Sequenz) wenigstens 3 Sequenzmotive ATTA (SEQ ID NO:1) und wenigstens ein Sequenzmotiv TAAATATTTTA (SEQ ID NO:3) pro 100 Nukleotide über eine Strecke von höchstens 200 Nukleotiden umfasst; vorzugsweise wenigstens 20 Sequenzmotive ATTA über eine Sequenz von 500 Nukleotiden umfasst,
wobei das Polynukleotid ferner eine codierende Sequenz umfasst, die einen selektierbaren Marker codiert (selektierbare Markersequenz), wobei sich die selektierbare Markersequenz zwischen dem Promotor und dem S/MAR-Element befindet, vorzugsweise wobei der Promotor und die selektierbare Markersequenz zusammen ein selektierbares Marker-Gen bilden.

2. Polynukleotid nach Anspruch 1, wobei es sich bei dem selektierbaren Marker um eine Nukleinsäuresequenz handelt, die bei ihrer Expression in einer Wirtszelle Resistenz gegen wenigstens eine Bedingung verleiht, durch die bei ihrer Anwendung auf eine Wirtszelle Selektionsdruck auf diese vermittelt wird.

3. Polynukleotid nach einem der Ansprüche 1 oder 2, wobei es sich bei dem selektierbaren Marker um einen selektierbaren Marker einer eukaryontischen Zelle handelt.

4. Polynukleotid nach einem der Ansprüche 1 bis 3, wobei es sich bei dem selektierbaren Marker-Gen um ein Puromycinresistenz-Gen, ein Blasticidinresistenz-Gen, ein Neomycinresistenz-Gen oder ein Zeocinresistenz-Gen, bevorzugt um ein Puromycinresistenz-Gen, handelt.

5. Polynukleotid nach einem der Ansprüche 1 bis 4, wobei das selektierbare Marker-Gen ohne ein Poly-A-Signal und Transkriptionsterminationssignal(e) vorliegt.

6. Polynukleotid nach einem der Ansprüche 1 bis 5, wobei das selektierbare Marker-Gen eine Nukleinsäuresequenz umfasst, die
a) die Expression eines Puromycinresistenz-Polypeptids, das die Sequenz unter SEQ ID NO:9 umfasst, veranlasst;
b) die Expression eines Puromycinresistenz-Polypeptids, das eine mit der Sequenz unter SEQ ID NO:9 zu wenigstens 70% identische Sequenz umfasst, veranlasst;
c) die Sequenz unter SEQ ID NO:10 umfasst;
d) eine mit der Sequenz unter SEQ ID NO:10 zu wenigstens 70% identische Sequenz umfasst,
e) eine Nukleinsäuresequenz umfasst, die ein Puromycinresistenz-Polypeptid codiert, das die Sequenz unter SEQ ID NO:9 umfasst bzw. bevorzugt daraus besteht, und/oder
f) eine Nukleinsäuresequenz umfasst, die ein Puromycinresistenz-Polypeptid codiert, das eine mit der Sequenz unter SEQ ID NO:9 zu wenigstens 70% identische Sequenz umfasst bzw. bevorzugt daraus besteht.

7. Polynukleotid nach einem der Ansprüche 1 bis 6, wobei die S/MAR-Sequenz unmittelbar stromabwärts vom Promotor und vom selektierbaren Marker-Gen liegt, vorzugsweise wobei unmittelbar stromabwärts liegend in einer solchen Nähe liegend bedeutet, dass Transkripte der selektierbaren Markersequenz eine transkribierte S/MAR-Sequenz umfassen.

8. Polynukleotid nach einem der Ansprüche 1 bis 7, wobei das Polynukleotid in einer Wirtszelle episomal repliziert, vorzugsweise wobei es sich bei der episomalen Replikation um stabile episomale Replikation handelt, bevorzugt in einer Säugerzelle.

9. Zusammensetzung, umfassend ein Polynukleotid gemäß einem der Ansprüche 1 bis 8, vorzugsweise wobei es sich bei der Zusammensetzung um eine pharmazeutische Zusammensetzung handelt.

10. Wirtszelle, umfassend ein Polynukleotid gemäß einem der Ansprüche 1 bis 8, vorzugsweise wobei es sich bei der Wirtszelle um eine CD34+-Progenitor-Zelle; einen CD61+-Thrombozyten; einen CD19+-B-Lymphozyten; einen CD14+-Monozyten; einen CD15+-Granulozyten; einen zytotoxischen CD3+-T-Lymphozyten, bevorzugt auch positiv für CD8 und CD45; einen CD3+-Helfer-T-Lymphozyten, bevorzugt auch positiv für CD4 und CD45; einen aktivierten CD3+-T-Lymphozyten, bevorzugt auch positiv für CD25 und CD45, einen Tumor-infiltrierenden Lymphozyten oder eine NK(Natural Killer)-Zelle handelt.

11. Polynukleotid gemäß einem der Ansprüche 1 bis 8, Zusammensetzung gemäß Anspruch 9 und/oder Wirtszelle gemäß Anspruch 10, zur medizinischen Verwendung, vorzugsweise zur Verwendung bei der Behandlung einer Erbkrankheit, bevorzugt monogenen Krankheit, stärker bevorzugt monogen rezessiven Krankheit, am stärksten bevorzugt von Phenylketonurie, Alkaptonurie, Leberscher kongenitaler Amaurose, Choroideremie oder Morbus Stargardt.

12. Kit, umfassend ein Polynukleotid gemäß einem der Ansprüche 1 bis 8 und eine den Zelleintritt vermittelnde Verbindung.

13. Vorrichtung, umfassend ein Polynukleotid gemäß einem der Ansprüche 1 bis 8, eine Zusammensetzung gemäß Anspruch 9 und/oder eine Wirtszelle gemäß Anspruch 10.

14. Verfahren zur stabilen Transfektion einer Wirtszelle, umfassend
a) Inkontaktbringen der Wirtszelle mit einem Polynukleotid gemäß einem der Ansprüche 1 bis 8, einer Zusammensetzung gemäß Anspruch 9 und/oder einer Wirtszelle gemäß Anspruch 10 und
b) dadurch stabiles Transfizieren einer Wirtszelle.

15. Verwendung eines Polynukleotids gemäß einem der Ansprüche 1 bis 8 zur stabilen gentechnischen Veränderung einer Wirtszelle.

## Revendications

1. Polynucléotide comprenant au moins un promoteur et un élément S/MAR, dans lequel ledit élément S/MAR est situé en aval dudit promoteur et dans lequel la séquence d'acide nucléique dudit élément S/MAR (séquence S/MAR) comprend au moins 3 motifs de séquence ATTA (SEQ ID NO : 1) et au moins un motif TAAATATTTTA (SEQ ID NO:3) par 100 nucléotides sur un segment d'au plus 200 nucléotides ; de préférence comprend au moins 20 motifs de séquence ATTA sur une séquence de 500 nucléotides,
ledit polynucléotide comprenant en outre une séquence codante codant pour un marqueur sélectionnable (séquence de marqueur sélectionnable), ladite séquence de marqueur sélectionnable étant intercalée entre ledit promoteur et ledit élément S/MAR, de préférence dans lequel ledit promoteur et ladite séquence de marqueur sélectionnable constituent conjointement un gène marqueur sélectionnable.

2. Polynucléotide selon la revendication 1, dans lequel ledit marqueur sélectionnable est une séquence d'acide nucléique conférant, lorsqu'il est exprimé dans une cellule hôte, une résistance à au moins une pression de sélection médiant une affection à une cellule hôte lorsqu'il est appliqué à celle-ci.

3. Polynucléotide selon l'une quelconque des revendications 1 ou 2, dans lequel le marqueur sélectionnable est un marqueur sélectionnable d'une cellule eucaryote.

4. Polynucléotide selon l'une quelconque des revendications 1 à 3, dans lequel ledit gène marqueur sélectionnable est un gène de résistance à la puromycine, un gène de résistance à la blasticidine, un gène de résistance à la néomycine, ou un gène de résistance à la zéocine, de préférence est un gène de résistance à la puromycine.

5. Polynucléotide selon l'une quelconque des revendications 1 à 4, dans lequel le gène marqueur sélectionnable est exempt d'un signal poly-A et de signal/signaux de terminaison de transcription.

6. Polynucléotide selon l'une quelconque des revendications 1 à 5, dans lequel le gène marqueur sélectionnable comprend une séquence d'acide nucléique qui
a) cause l'expression d'un polypeptide de résistance à la puromycine comprenant la séquence de SEQ ID NO : 9 ;
b) cause l'expression d'un polypeptide de résistance à la puromycine comprenant une séquence au moins 70 % identique à la séquence de SEQ ID NO : 9 ;
c) comprend la séquence de SEQ ID NO : 10 ;
d) comprend une séquence au moins 70 % identique à la séquence de SEQ ID NO : 10,
e) comprend une séquence d'acide nucléique codant pour un polypeptide de résistance à la puromycine comprenant, de préférence constitué de, la séquence de SEQ ID NO : 9, et/ou
f) comprend une séquence d'acide nucléique codant pour un polypeptide de résistance à la puromycine comprenant, de préférence constitué de, une séquence au moins 70 % identique à la séquence de SEQ ID NO : 9.

7. Polynucléotide selon l'une quelconque des revendications 1 à 6, dans lequel ladite séquence S/MAR est située immédiatement en aval dudit promoteur et dudit gène marqueur sélectionnable, de préférence étant située immédiatement en aval signifie située à une proximité telle que les produits de transcription de la séquence de marqueur sélectionnable comprennent une séquence S/MAR transcrite.

8. Polynucléotide selon l'une quelconque des revendications 1 à 7, ledit polynucléotide se répliquant de façon épisomique dans une cellule hôte, la réplication épisomique étant de préférence une réplication épisomique stable, de préférence dans une cellule de mammifère.

9. Composition comprenant un polynucléotide selon l'une quelconque des revendications 1 à 8, ladite composition étant de préférence une composition pharmaceutique.

10. Cellule hôte comprenant le polynucléotide selon l'une quelconque des revendications 1 à 8, ladite cellule hôte étant de préférence un cellule progénitrice CD34+ ; un thrombocyte CD61+ ; un lymphocyte B CD19+ ; un monocyte CD14+ ; un granulocyte CD15+ ; un lymphocyte T cytotoxique CD3+, de préférence également positif pour CD8 et CD45 ; un lymphocyte T auxiliaire CD3+, de préférence également positif pour CD4 et CD45 ; un lymphocyte T activé CD3+, de préférence également positif pour CD25 et CD45, un lymphocyte infiltrant une tumeur, ou une cellule tueuse naturelle (NK).

11. Polynucléotide selon l'une quelconque des revendications 1 à 8, composition selon la revendication 9, et/ou cellule hôte selon la revendication 10, pour utilisation en médecine, de préférence, pour utilisation dans le traitement d'une maladie génétique, de préférence une maladie monogénique, plus préférablement une maladie monogénique récessive, de manière préférée entre toutes la phénylcétonurie, l'alcaptonurie, l'amaurose congénitale de Leber, la choroïdérémie ou la maladie de Stargardt.

12. Kit comprenant un polynucléotide selon l'une quelconque des revendications 1 à 8 et un composé médiant l'entrée cellulaire.

13. Dispositif comprenant un polynucléotide selon l'une quelconque des revendications 1 à 8, une composition selon la revendication 9, et/ou une cellule hôte selon la revendication 10.

14. Procédé pour transfecter de façon stable une cellule hôte, comprenant
a) la mise en contact de ladite cellule hôte avec un polynucléotide selon l'une quelconque des revendications 1 à 8, une composition selon la revendication 9, et/ou une cellule hôte selon la revendication 10, et,
b) de façon à transfecter de façon stable une cellule hôte.

15. Utilisation d'un polynucléotide selon l'une quelconque des revendications 1 à 8 pour modifier génétiquement de façon stable une cellule hôte.


### SEQUENCE LISTING

<110> Deutsches Krebsforschungszentrum
<120> Non-Integrating DNA Vectors for the genetic modification of Cells
<130> DK14791EP
<160> 14
<170> PatentIn version 3.3
<210> 1
   <211> 4
   <212> DNA
   <213> Homo sapiens
<400> 1
   atta 4
<210> 2
   <211> 5
   <212> DNA
   <213> Homo sapiens
<400> 2
   attta 5
<210> 3
   <211> 11
   <212> DNA
   <213> Homo sapiens
<400> 3
   taaatatttt a 11
<210> 4
   <211> 19
   <212> DNA
   <213> Homo sapiens
<400> 4
   attataaata ttttaatta 19
<210> 5
   <211> 24
   <212> DNA
   <213> Homo sapiens
<400> 5
   atttaattat aaatatttta atta 24
<210> 6
   <211> 525
   <212> DNA
   <213> Homo sapiens
<400> 6
   atttataaaa tattgaatta taaaatatgt aattataaat actttaatta taaaatatgt     60
   aattataaat actttaatta taaaatatgt aattataaat actttataaa atatgtaatt    120
   ataaaatatg taattataaa cattttaatt ataaaatatg taattataaa cattttaatt    180
   ataaaatatg taattataaa cattttaatt ataaaatatg taattataaa cattttaatt    240
   ataaaatatg taattataaa cattttaatt ataaaatatt taattataaa cattttaatt    300
   ataaaatatt taattataaa tattttaatt ataaaatatt taattataaa tattttaatt    360
   ataaaatatt taattataaa tattttaatt ataaaatatt taattataaa tactttaatt    420
   ataaaatatt taattataaa tattttaatt ataaaatatt taattataaa tattttaatt    480
   ataaatattt taattataaa atatttaatt ataaaaacac aatta                    525
<210> 7
   <211> 825
   <212> DNA
   <213> Homo sapiens
<400> 7
   gcaggctgag tgaaataaag gacttgttat ttcatctcga ggcctaccgg agagccttgc     60
   cttgcaaagg cagacagtca gtgaggaaga ctatgtggca catgaagaca ccagaggtgt    120
   tcctcaggat caaagtatgt acaagccttt gtgaatattt tttccttctc acttggcaaa    180
   tacaattcct gagatcaata acctcgtctt tttaattttt tcctcgtctt tttaactatt    240
   tataaaatat tgaattataa aatatgtaat tataaatact ttaattataa aatatgtaat    300
   tataaatact ttaattataa aatatgtaat tataaatact ttataaaata tgtaattata    360
   aaatatgtaa ttataaacat tttaattata aaatatgtaa ttataaacat tttaattata    420
   aaatatgtaa ttataaacat tttaattata aaatatgtaa ttataaacat tttaattata    480
   aaatatgtaa ttataaacat tttaattata aaatatttaa ttataaacat tttaattata    540
   aaatatttaa ttataaatat tttaattata aaatatttaa ttataaatat tttaattata    600
   aaatatttaa ttataaatat tttaattata aaatatttaa ttataaatac tttaattata    660
   aaatatttaa ttataaatat tttaattata aaatatttaa ttataaatat tttaattata    720
   aatattttaa ttataaaata tttaattata aaaacacaat tacctcatct ttttaaatat    780
   ttttgcaaaa tatttccctc cataatttct ccgtttccat tttta                    825
<210> 8
   <211> 854
   <212> DNA
   <213> Homo sapiens
<400> 8
   cttctccact cctggcaggc tgagtgaaat aaaggacttg ttatttcatc tcgaggccta    60
   ccggagagcc ttgccttgca aaggcagaca gtcagtgagg aagactatgt ggcacatgaa   120
   gacaccagag gtgttcctca ggatcaaagt atgtacaagc ctttgtgaat attttttcct   180
   tctcacttgg caaatacaat tcctgagatc aataacctcg tctttttaat tttttcctcg   240
   tctttttaac tatttataaa atattgaatt ataaaatatg taattataaa tactttaatt   300
   ataaaatatg taattataaa tactttaatt ataaaatatg taattataaa tactttataa   360
   aatatgtaat tataaaatat gtaattataa acattttaat tataaaatat gtaattataa   420
   acattttaat tataaaatat gtaattataa acattttaat tataaaatat gtaattataa   480
   acattttaat tataaaatat gtaattataa acattttaat tataaaatat ttaattataa   540
   acattttaat tataaaatat ttaattataa atattttaat tataaaatat ttaattataa   600
   atattttaat tataaaatat ttaattataa atattttaat tataaaatat ttaattataa   660
   atactttaat tataaaatat ttaattataa atattttaat tataaaatat ttaattataa   720
   atattttaat tataaatatt ttaattataa aatatttaat tataaaaaca caattacctc   780
   atctttttaa atatttttgc aaaatatttc cctccataat ttctccgttt ccatttttat   840
   tctgttactt aaat                                                     854
<210> 9
   <211> 199
   <212> PRT
   <213> Artificial
<220>
   <223> puromycin acetyltransferase, synthetic construct
<400> 9
   Met Thr Glu Tyr Lys Pro Thr Val Arg Leu Ala Thr Arg Asp Asp Val
   1               5                   10                  15
   Pro Arg Ala Val Arg Thr Leu Ala Ala Ala Phe Ala Asp Tyr Pro Ala
               20                  25                  30
   Thr Arg His Thr Val Asp Pro Asp Arg His Ile Glu Arg Val Thr Glu
           35                  40                  45
   Leu Gln Glu Leu Phe Leu Thr Arg Val Gly Leu Asp Ile Gly Lys Val
       50                  55                  60
   Trp Val Ala Asp Asp Gly Ala Ala Val Ala Val Trp Thr Thr Pro Glu
   65                  70                  75                  80
   Ser Val Glu Ala Gly Ala Val Phe Ala Glu Ile Gly Pro Arg Met Ala
                   85                  90                  95
   Glu Leu Ser Gly Ser Arg Leu Ala Ala Gln Gln Gln Met Glu Gly Leu
               100                 105                 110
   Leu Ala Pro His Arg Pro Lys Glu Pro Ala Trp Phe Leu Ala Thr Val
           115                 120                 125
   Gly Val Ser Pro Asp His Gln Gly Lys Gly Leu Gly Ser Ala Val Val
       130                 135                 140
   Leu Pro Gly Val Glu Ala Ala Glu Arg Ala Gly Val Pro Ala Phe Leu
   145                 150                 155                 160
   Glu Thr Ser Ala Pro Arg Asn Leu Pro Phe Tyr Glu Arg Leu Gly Phe
                   165                 170                 175
   Thr Val Thr Ala Asp Val Glu Val Pro Glu Gly Pro Arg Thr Trp Cys
               180                 185                 190
   Met Thr Arg Lys Pro Gly Ala
           195
<210> 10
   <211> 600
   <212> DNA
   <213> Artificial
<220>
   <223> puromycin acetyltransferase encoding sequence, synthetic construct
<400> 10
   atgaccgagt acaagcccac ggtgcgcctc gccacccgcg acgacgtccc ccgggccgta     60
   cgcaccctcg ccgccgcgtt cgccgactac cccgccacgc gccacaccgt cgacccggac    120
   cgccacatcg agcgggtcac cgagctgcaa gaactcttcc tcacgcgcgt cgggctcgac    180
   atcggcaagg tgtgggtcgc ggacgacggc gccgcggtgg cggtctggac cacgccggag    240
   agcgtcgaag cgggggcggt gttcgccgag atcggcccgc gcatggccga gttgagcggt    300
   tcccggctgg ccgcgcagca acagatggaa ggcctcctgg cgccgcaccg gcccaaggag    360
   cccgcgtggt tcctggccac cgtcggcgtc tcgcccgacc accagggcaa gggtctgggc    420
   agcgccgtcg tgctccccgg agtggaggcg gccgagcgcg ccggggtgcc cgccttcctg    480
   gagacctccg cgccccgcaa cctccccttc tacgagcggc tcggcttcac cgtcaccgcc    540
   gacgtcgagg tgcccgaagg accgcgcacc tggtgcatga cccgcaagcc cggtgcctga    600
<210> 11
   <211> 1031
   <212> DNA
   <213> Artificial
<220>
   <223> anti-repressive element40
<400> 11
   gatcaagaaa gcactccggg ctccagaagg agccttccag gccagctttg agcataagct      60
   gctgatgagc agtgagtgtc ttgagtagtg ttcagggcag catgttacca ttcatgcttg     120
   acttctagcc agtgtgacga gaggctggag tcaggtctct agagagttga gcagctccag     180
   ccttagatct cccagtctta tgcggtgtgc ccattcgctt tgtgtctgca gtcccctggc     240
   cacacccagt aacagttctg ggatctatgg gagtagcttc cttagtgagc tttcccttca     300
   aatactttgc aaccaggtag agaagtttgg agtgaaggtt ttgttcttcg tttcttcaca     360
   atatggatat gcatcttctt ttgaaaatgt taaagtaaat tacctctctt ttcagatact     420
   gtcttcatgc gaacttggta tcctgtttcc atcccagcct tctataaccc agtaacatct     480
   tttttgaaac cagtgggtga gaaagacacc tggtcaggaa cgcggaccac aggacaactc     540
   aggctcaccc acggcatcag actaaaggca aacaaggact ctgtataaag taccggtggc     600
   atgtgtatta gtggagatgc agcctgtgct ctgcagacag ggagtcacac agacactttt     660
   ctataatttc ttaagtgctt tgaatgttca agtagaaagt ctaacattaa atttgattga     720
   acaattgtat attcatggaa tattttggaa cggaatacca aaaaatggca atagtggttc     780
   tttctggatg gaagacaaac ttttcttctt taaaataaat tttattttat atatttgagg     840
   ttgaccacat gaccttaagg atacatatag acagtaaact ggttactaca gtgaagcaaa     900
   ttaacatatc taccatcgta catagttaca tttttttgtg tgacaggaac agctaaaatc     960
   tacgtattta acaaaactcc taaagacaat acatttttat taactatagc cctcatgatg    1020
   tacattagat c                                                         1031
<210> 12
   <211> 1438
   <212> DNA
   <213> Artificial
<220>
   <223> CMV Promoter - S/MAR sequence
<400> 12
   ggcattgatt attgactagt tattaatagt aatcaattac ggggtcatta gttcatagcc      60
   catatatgga gttccgcgtt acataactta cggtaaatgg cccgcctggc tgaccgccca     120
   acgacccccg cccattgacg tcaataatga cgtatgttcc catagtaacg ccaataggga     180
   ctttccattg acgtcaatgg gtggagtatt tacggtaaac tgcccacttg gcagtacatc     240
   aagtgtatca tatgccaagt ccgcccccta ttgacgtcaa tgacggtaaa tggcccgcct     300
   ggcattatgc ccagtacatg accttacggg actttcctac ttggcagtac atctacgtat     360
   tagtcatcgc tattaccatg gtgatgcggt tttggcagta caccaatggg cgtggatagc     420
   ggtttgactc acggggattt ccaagtctcc accccattga cgtcaatggg agtttgtttt     480
   ggcaccaaaa tcaacgggac tttccaaaat gtcgtaataa ccccgccccg ttgacgcaaa     540
   tgggcggtag gcgtgtacgg tgggaggtct atataagcag aggtcttctc cactcctggc     600
   aggctgagtg aaataaagga cttgttattt catctcgagg cctaccggag agccttgcct     660
   tgcaaaggca gacagtcagt gaggaagact atgtggcaca tgaagacacc agaggtgttc     720
   ctcaggatca aagtatgtac aagcctttgt gaatattttt tccttctcac ttggcaaata     780
   caattcctga gatcaataac ctcgtctttt taattttttc ctcgtctttt taactattta     840
   taaaatattg aattataaaa tatgtaatta taaatacttt aattataaaa tatgtaatta     900
   taaatacttt aattataaaa tatgtaatta taaatacttt ataaaatatg taattataaa     960
   atatgtaatt ataaacattt taattataaa atatgtaatt ataaacattt taattataaa    1020
   atatgtaatt ataaacattt taattataaa atatgtaatt ataaacattt taattataaa    1080
   atatgtaatt ataaacattt taattataaa atatttaatt ataaacattt taattataaa    1140
   atatttaatt ataaatattt taattataaa atatttaatt ataaatattt taattataaa    1200
   atatttaatt ataaatattt taattataaa atatttaatt ataaatactt taattataaa    1260
   atatttaatt ataaatattt taattataaa atatttaatt ataaatattt taattataaa    1320
   tattttaatt ataaaatatt taattataaa aacacaatta cctcatcttt ttaaatattt    1380
   ttgcaaaata tttccctcca taatttctcc gtttccattt ttattctgtt acttaaat      1438
<210> 13
   <211> 2038
   <212> DNA
   <213> Artificial
<220>
   <223> CMV Promoter - Puromycin - S/MAR sequence
<400> 13
   ggcattgatt attgactagt tattaatagt aatcaattac ggggtcatta gttcatagcc     60
   catatatgga gttccgcgtt acataactta cggtaaatgg cccgcctggc tgaccgccca    120
   acgacccccg cccattgacg tcaataatga cgtatgttcc catagtaacg ccaataggga    180
   ctttccattg acgtcaatgg gtggagtatt tacggtaaac tgcccacttg gcagtacatc    240
   aagtgtatca tatgccaagt ccgcccccta ttgacgtcaa tgacggtaaa tggcccgcct    300
   ggcattatgc ccagtacatg accttacggg actttcctac ttggcagtac atctacgtat    360
   tagtcatcgc tattaccatg gtgatgcggt tttggcagta caccaatggg cgtggatagc    420
   ggtttgactc acggggattt ccaagtctcc accccattga cgtcaatggg agtttgtttt    480
   ggcaccaaaa tcaacgggac tttccaaaat gtcgtaataa ccccgccccg ttgacgcaaa    540
   tgggcggtag gcgtgtacgg tgggaggtct atataagcag aggtatgacc gagtacaagc    600
   ccacggtgcg cctcgccacc cgcgacgacg tcccccgggc cgtacgcacc ctcgccgccg    660
   cgttcgccga ctaccccgcc acgcgccaca ccgtcgaccc ggaccgccac atcgagcggg    720
   tcaccgagct gcaagaactc ttcctcacgc gcgtcgggct cgacatcggc aaggtgtggg    780
   tcgcggacga cggcgccgcg gtggcggtct ggaccacgcc ggagagcgtc gaagcggggg    840
   cggtgttcgc cgagatcggc ccgcgcatgg ccgagttgag cggttcccgg ctggccgcgc    900
   agcaacagat ggaaggcctc ctggcgccgc accggcccaa ggagcccgcg tggttcctgg    960
   ccaccgtcgg cgtctcgccc gaccaccagg gcaagggtct gggcagcgcc gtcgtgctcc   1020
   ccggagtgga ggcggccgag cgcgccgggg tgcccgcctt cctggagacc tccgcgcccc   1080
   gcaacctccc cttctacgag cggctcggct tcaccgtcac cgccgacgtc gaggtgcccg   1140
   aaggaccgcg cacctggtgc atgacccgca agcccggtgc ctgacttctc cactcctggc   1200
   aggctgagtg aaataaagga cttgttattt catctcgagg cctaccggag agccttgcct   1260
   tgcaaaggca gacagtcagt gaggaagact atgtggcaca tgaagacacc agaggtgttc   1320
   ctcaggatca aagtatgtac aagcctttgt gaatattttt tccttctcac ttggcaaata   1380
   caattcctga gatcaataac ctcgtctttt taattttttc ctcgtctttt taactattta   1440
   taaaatattg aattataaaa tatgtaatta taaatacttt aattataaaa tatgtaatta   1500
   taaatacttt aattataaaa tatgtaatta taaatacttt ataaaatatg taattataaa   1560
   atatgtaatt ataaacattt taattataaa atatgtaatt ataaacattt taattataaa   1620
   atatgtaatt ataaacattt taattataaa atatgtaatt ataaacattt taattataaa   1680
   atatgtaatt ataaacattt taattataaa atatttaatt ataaacattt taattataaa   1740
   atatttaatt ataaatattt taattataaa atatttaatt ataaatattt taattataaa   1800
   atatttaatt ataaatattt taattataaa atatttaatt ataaatactt taattataaa   1860
   atatttaatt ataaatattt taattataaa atatttaatt ataaatattt taattataaa   1920
   tattttaatt ataaaatatt taattataaa aacacaatta cctcatcttt ttaaatattt   1980
   ttgcaaaata tttccctcca taatttctcc gtttccattt ttattctgtt acttaaat     2038
<210> 14
   <211> 6189
   <212> DNA
   <213> Artificial
<220>
   <223> Element40-GPF-P2A-Puromycin-S/MAR
<400> 14
   ggcattgatt attgactagt tattaatagt aatcaattac ggggtcatta gttcatagcc      60
   catatatgga gttccgcgtt acataactta cggtaaatgg cccgcctggc tgaccgccca     120
   acgacccccg cccattgacg tcaataatga cgtatgttcc catagtaacg ccaataggga 180
   ctttccattg acgtcaatgg gtggagtatt tacggtaaac tgcccacttg gcagtacatc 240
   aagtgtatca tatgccaagt ccgcccccta ttgacgtcaa tgacggtaaa tggcccgcct 300
   ggcattatgc ccagtacatg accttacggg actttcctac ttggcagtac atctacgtat 360
   tagtcatcgc tattaccatg gtgatgcggt tttggcagta caccaatggg cgtggatagc 420
   ggtttgactc acggggattt ccaagtctcc accccattga cgtcaatggg agtttgtttt 480
   ggcaccaaaa tcaacgggac tttccaaaat gtcgtaataa ccccgccccg ttgacgcaaa 540
   tgggcggtag gcgtgtacgg tgggaggtct atataagcag aggtcgttta gtgaaccgtc 600
   agatcactag tagctttatt gcggtagttt atcacagtta aattgctaac gcagtcagtg 660
   ctcgactgat cacaggtaag tatcaaggtt acaagacagg tttaaggagg ccaatagaaa 720
   ctgggcttgt cgagacagag aagattcttg cgtttctgat aggcacctat tggtcttact 780
   gacatccact ttgcctttct ctccacaggg gtaccgaagc cgctagcgct accggtcgcc 840
   accatgcccg ccatgaagat cgagtgccgc atcaccggca ccctgaacgg cgtggagttc 900
   gagctggtgg gcggcggaga gggcaccccc gagcagggcc gcatgaccaa caagatgaag 960
   agcaccaaag gcgccctgac cttcagcccc tacctgctga gccacgtgat gggctacggc 1020
   ttctaccact tcggcaccta ccccagcggc tacgagaacc ccttcctgca cgccatcaac 1080
   aacggcggct acaccaacac ccgcatcgag aagtacgagg acggcggcgt gctgcacgtg 1140
   agcttcagct accgctacga ggccggccgc gtgatcggcg acttcaaggt ggtgggcacc 1200
   ggcttccccg aggacagcgt gatcttcacc gacaagatca tccgcagcaa cgccaccgtg 1260
   gagcacctgc accccatggg cgataacgtg ctggtgggca gcttcgcccg caccttcagc 1320
   ctgcgcgacg gcggctacta cagcttcgtg gtggacagcc acatgcactt caagagcgcc 1380
   atccacccca gcatcctgca gaacgggggc cccatgttcg ccttccgccg cgtggaggag 1440
   ctgcacagca acaccgagct gggcatcgtg gagtaccagc acgccttcaa gacccccatc 1500
   gccttcgcca gatctggcag cggcgccacc aacttcagcc tgctgaagca ggccggcgac 1560
   gtggaggaaa accctgggcc catgaccgag tacaagccca cggtgcgcct cgccacccgc 1620
   gacgacgtcc cccgggccgt acgcaccctc gccgccgcgt tcgccgacta ccccgccacg 1680
   cgccacaccg tcgacccgga ccgccacatc gagcgggtca ccgagctgca agaactcttc 1740
   ctcacgcgcg tcgggctcga catcggcaag gtgtgggtcg cggacgacgg cgccgcggtg 1800
   gcggtctgga ccacgccgga gagcgtcgaa gcgggggcgg tgttcgccga gatcggcccg 1860
   cgcatggccg agttgagcgg ttcccggctg gccgcgcagc aacagatgga aggcctcctg 1920
   gcgccgcacc ggcccaagga gcccgcgtgg ttcctggcca ccgtcggcgt ctcgcccgac 1980
   caccagggca agggtctggg cagcgccgtc gtgctccccg gagtggaggc ggccgagcgc 2040
   gccggggtgc ccgccttcct ggagacctcc gcgccccgca acctcccctt ctacgagcgg 2100
   ctcggcttca ccgtcaccgc cgacgtcgag gtgcccgaag gaccgcgcac ctggtgcatg 2160
   acccgcaagc ccggtgcctg aagatctatg catgcagaag ttggtcgtga ggcactgggc 2220
   aggtaagtat caaggttaca agacaggtcg acttctccac tcctggcagg ctgagtgaaa 2280
   taaaggactt gttatttcat ctcgaggcct accggagagc cttgccttgc aaaggcagac 2340
   agtcagtgag gaagactatg tggcacatga agacaccaga ggtgttcctc aggatcaaag 2400
   tatgtacaag cctttgtgaa tattttttcc ttctcacttg gcaaatacaa ttcctgagat 2460
   caataacctc gtctttttaa ttttttcctc gtctttttaa ctatttataa aatattgaat 2520
   tataaaatat gtaattataa atactttaat tataaaatat gtaattataa atactttaat 2580
   tataaaatat gtaattataa atactttata aaatatgtaa ttataaaata tgtaattata 2640
   aacattttaa ttataaaata tgtaattata aacattttaa ttataaaata tgtaattata 2700
   aacattttaa ttataaaata tgtaattata aacattttaa ttataaaata tgtaattata 2760
   aacattttaa ttataaaata tttaattata aacattttaa ttataaaata tttaattata 2820
   aatattttaa ttataaaata tttaattata aatattttaa ttataaaata tttaattata 2880
   aatattttaa ttataaaata tttaattata aatactttaa ttataaaata tttaattata 2940
   aatattttaa ttataaaata tttaattata aatattttaa ttataaatat tttaattata 3000
   aaatatttaa ttataaaaac acaattacct catcttttta aatatttttg caaaatattt 3060
   ccctccataa tttctccgtt tccattttta ttctgttact taaataattc tgcagtcgac 3120
   ggttactgac atccactttg cctttctctc cacaggtgtc cactctaccg cgggcccggg 3180
   atccaccgga tctagataac tgatcataat cagccatacc acatttgtag aggttttact 3240
   tgctttaaaa aacctcccac acctccccct gaacctgaaa cataaaatga atgcaattgt 3300
   tgttgttaac ttgtttattg cagcttataa tggttacaaa taaagcaata gcatcacaaa 3360
   tttcacaaat aaagcatttt tttcactgca ttctagttgt ggtttgtcca aactcatcaa 3420
   tgtatcttac atgtgagcaa aaggccagca aaaggccagg aaccgtaaaa aggccgcgtt 3480
   gctggcgttt ttccataggc tccgcccccc tgacgagcat cacaaaaatc gacgctcaag 3540
   tcagaggtgg cgaaacccga caggactata aagataccag gcgtttcccc ctggaagctc 3600
   cctcgtgcgc tctcctgttc cgaccctgcc gcttaccgga tacctgtccg cctttctccc 3660
   ttcgggaagc gtggcgcttt ctcatagctc acgctgtagg tatctcagtt cggtgtaggt 3720
   cgttcgctcc aagctgggct gtgtgcacga accccccgtt cagcccgacc gctgcgcctt 3780
   atccggtaac tatcgtcttg agtccaaccc ggtaagacac gacttatcgc cactggcagc 3840
   agccactggt aacaggatta gcagagcgag gtatgtaggc ggtgctacag agttcttgaa 3900
   gtggtggcct aactacggct acactagaag aacagtattt ggtatctgcg ctctgctgaa 3960
   gccagttacc ttcggaaaaa gagttggtag ctcttgatcc ggcaaacaaa ccaccgctgg 4020
   tagcggtggt ttttttgttt gcaagcagca gattacgcgc agaaaaaaag gatctcaaga 4080
   agatcctttg atcttttcta cggggtctga cgctcagtgg aacgaaaact cacgttaagg 4140
   gattttggtc atgccgtctc agaagaactc gtcaagaagg cgatagaagg cgatgcgctg 4200
   cgaatcggga gcggcgatac cgtaaagcac gaggaagcgg tcagcccatt cgccgccaag 4260
   ctcttcagca atatcacggg tagccaacgc tatgtcctga tagcggtccg ccacacccag 4320
   ccggccacag tcgatgaatc cagaaaagcg gccattttcc accatgatat tcggcaagca 4380
   ggcatcgcca tgggtcacga cgagatcctc gccgtcgggc atgctcgcct tgagcctggc 4440
   gaacagttcg gctggcgcga gcccctgatg ctcttcgtcc agatcatcct gatcgacaag 4500
   accggcttcc atccgagtac gtgctctctc gatgcgatgt ttcgcttggt ggtcgaatgg 4560
   gcaggtagcc ggatcaagcg tatgcagccg ccgcattgca tcagccatga tggatacttt 4620
   ctcggcagga gcaaggtgag atgacaggag atcctgcccc ggcacttcgc ccaatagcag 4680
   ccagtccctt cccgcttcag tgacaacgtc gagtacagct gcgcaaggaa cgcccgtcgt 4740
   ggccagccac gatagccgcg ctgcctcgtc ttgcagttca ttcagggcac cggacaggtc 4800
   ggtcttgaca aaaagaaccg ggcgcccctg cgctgacagc cggaacacgg cggcatcaga 4860
   gcagccgatt gtctgttgtg cccagtcata gccgaatagc ctctccaccc aagcggccgg 4920
   agaacctgcg tgcaatccat cttgttcaat cataatatta ttgaagcatt tatcagggtt 4980
   cgtctcgtcc cggtctcctc ccatgcatgt caatattggc cattagccat attattcatt 5040
   ggttatatag cataaatcaa tattggctat tggccattgc atacgttgta tctatatcat 5100
   aatatgtaca aagcttgatc aagaaagcac tccgggctcc agaaggagcc ttccaggcca 5160
   gctttgagca taagctgctg atgagcagtg agtgtcttga gtagtgttca gggcagcatg 5220
   ttaccattca tgcttgactt ctagccagtg tgacgagagg ctggagtcag gtctctagag 5280
   agttgagcag ctccagcctt agatctccca gtcttatgcg gtgtgcccat tcgctttgtg 5340
   tctgcagtcc cctggccaca cccagtaaca gttctgggat ctatgggagt agcttcctta 5400
   gtgagctttc ccttcaaata ctttgcaacc aggtagagaa gtttggagtg aaggttttgt 5460
   tcttcgtttc ttcacaatat ggatatgcat cttcttttga aaatgttaaa gtaaattacc 5520
   tctcttttca gatactgtct tcatgcgaac ttggtatcct gtttccatcc cagccttcta 5580
   taacccagta acatcttttt tgaaaccagt gggtgagaaa gacacctggt caggaacgcg 5640
   gaccacagga caactcaggc tcacccacgg catcagacta aaggcaaaca aggactctgt 5700
   ataaagtacc ggtggcatgt gtattagtgg agatgcagcc tgtgctctgc agacagggag 5760
   tcacacagac acttttctat aatttcttaa gtgctttgaa tgttcaagta gaaagtctaa   5820
   cattaaattt gattgaacaa ttgtatattc atggaatatt ttggaacgga ataccaaaaa   5880
   atggcaatag tggttctttc tggatggaag acaaactttt cttctttaaa ataaatttta   5940
   ttttatatat ttgaggttga ccacatgacc ttaaggatac atatagacag taaactggtt   6000
   actacagtga agcaaattaa catatctacc atcgtacata gttacatttt tttgtgtgac   6060
   aggaacagct aaaatctacg tatttaacaa aactcctaaa gacaatacat ttttattaac   6120
   tatagccctc atgatgtaca ttagatctgt acatttatat tggctcatgt ccaatatgac   6180
   cgccatgtt                                                           6189